Europäisches Patentamt

European Patent Office

Office européen des brevets

(19)

(11) **EP 1 619 527 A1**

(12)

# EUROPEAN PATENT APPLICATION
published in accordance with Art. 158(3) EPC

(43) Date of publication:
**25.01.2006 Bulletin 2006/04**

(21) Application number: **04728084.7**

(22) Date of filing: **16.04.2004**

(51) Int Cl.:
*G02B 6/36* (1985.01)    *A61B 1/07* (1995.01)

(86) International application number:
**PCT/RU2004/000144**

(87) International publication number:
**WO 2004/092796 (28.10.2004 Gazette 2004/44)**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **17.04.2003 RU 2003111113**

(71) Applicant: **Imalux Corporation .**
**Cleveland, Ohio 44114 (US)**

(72) Inventors:
• **FELDCHTEIN, Felix Isaakovich**
**Cleveland, OH 44143 (US)**
• **GELIKONOV, Valentin Mikhailovich**
**Nizhny Novgorog, 603163 (RU)**
• **GELIKONOV, Grigory Valentinovich**
**Nizhny Novgorod, 603163 (RU)**

(74) Representative: **Roos, Peter et al**
**Klinger & Kollegen**
**Bavariaring 20**
**D-80336 München (DE)**

(54) **PROTECTOR FOR A FIBRE-OPTIC CATHETER**

(57)    The invention presents modifications of a protector design for an optical fiber probe intended for studying an object. The object being studied can be a biological tissue, namely, a biological tissue of a living body, for example, an internal cavity of a living body. The invention ensures an effective optical contact between an end face of a distal part of the optical fiber probe and the object being studied. In a preferred embodiment the later is achieved by designing an inner surface of a protector window capable of forming a temporary adhesive contact with the end face of the distal part of the optical fiber probe under a pressure of an axial force exerted on the optical fiber probe placed inside a sheath. Herewith, an outer surface of the protector window is designed capable of forming a temporary adhesive contact with the object being studied under the pressure of the axial force exerted on the optical fiber probe placed inside the sheath. To accomplish this in one embodiment the protector window is made of a pliable and resilient material, for example, of a cured optical gel. In another embodiment the protector window is configured as at least a bilayer structure. Additionally, in a preferred embodiment the layers, one of whose surfaces form either the inner or the outer surface of the protector window, are made of a pliable and resilient material, such as a cured optical gel. This prevents the protector window from sliding over the surface of the object being studied and at the same time ensures an effective optical contact between the end face of the distal part of the optical fiber probe and the object being studied. The cured optical gel can be jelly-like or rubber-like. The values of the refractive indexes of the protector window material at the operating wavelength or at least of the layer facing the interior cavity of the sheath and of the layer, one of whose surfaces forms the outer surface of the protector window, are chosen taking into account the values of refractive indexes of the distal part of the optical fiber probe and of the object being studied.

**EP 1 619 527 A1**

FIG. 2

## Description

### Technical field

The present invention relates to physical engineering, in particular, to the study of the internal structure of objects by optical means, and can be used, for example, in low coherence reflectometers, in devices for optical coherence tomography, in spectral imaging devices applied for medical diagnostics of individual organs and systems including *in vivo* or *in vitro* diagnostics, as well as for industrial diagnostics such as control of technological processes.

### Background art

Noninvasive diagnostics has become lately an object of great interest in medical practice, especially for studying internal organs of a living body, because of its obvious advantages over traditional biopsy surgery. Noninvasive diagnostics became possible due to the development of devices based on delivering optical radiation to a biological tissue, collecting the optical radiation reflected or backscattered by it, and subsequent processing the informative signal and imaging the biological tissue. The optical radiation is delivered by an optical fiber probe which is brought into contact with the biological tissue. Instruments used for *in vivo* medical studies must agree with sterility and disinfection requirements for the patient's safety. However, making the optical probe disposable might be economically impractical due to its high cost. Cleaning and disinfecting and/or sterilizing the probe prior to using it for a patient is time consuming, requires special equipment and shortens the time of effective use and the service life of the probe. In other cases, for example, for conducting studies *in vitro* or industrial diagnostics in aggressive mediums, it is necessary to take safety measures for the personnel working with the optical fiber probe and for protecting the probe itself from its being exposed to the test medium.

A solution to this problem was found by designing special optical fiber probe protectors, which cost much less than the optical fiber probe. That allows for making the protectors reusable after appropriate treatment, as well as disposable.

A prior art protector for an optical fiber probe used for studying biological tissues, according to U.S. Pat. No. 5,771,327 comprises a hollow sheath of a tubular configuration. In a preferred embodiment the sheath is fabricated from a rigid material such as plastic. A distal end of the sheath is equipped with a window, a proximal end of the sheath being interfaced with a hollow handle. The interior cavities of the sheath and the handle form a common space for placing the optical fiber probe in a position that the end face of the distal part of the optical fiber probe rests flatly on the inside surface of the protector window. The protector window is made of a rigid optically transparent material. In a preferred embodiment the handle includes a locking means for locking the optical fiber probe in the position. Nevertheless, there exists an air gap between the inner surface of the protector window and the end face of the distal part of the optical fiber probe due to rigidity of the material the protector window is made of and unevenness of its surface. For that reason the optical contact between the end face of the distal part of the optical fiber probe and the rigid protector window is not efficient and hence the optical contact between the end face of the distal part of the optical fiber probe and the biological tissue is not effective too.

In the protector according to U.S. Pat. No. 5,930,440 of an analogous design the rigid protector window is replaced by a pliable membrane made from polyurethane. The inventors believed that under a force applied to the optical fiber probe its end face would abut against the pliable membrane and as a result the optical contact between the end face of the distal part of the optical fiber probe and the pliable membrane would be effective. However, experience has shown that when using this protector power loses of the optical radiation may be considerably high. Besides, the optical radiation reflected or backscattered from the end face of the distal part of the optical fiber probe and from the inner surface of the protector window may be several orders of magnitude higher than the friendly optical signal reflected or backscattered from the biological tissue. That leads to additional optical losses and artifacts in measurements provided by an optical device such as, for example, a device for optical coherence tomography. The later is due to the fact that, as well known, in order to maintain an effective optical contact between two surfaces the air gap between them must be essentially smaller (for at least an order of magnitude) than the optical radiation wavelength. It is evident that the use of a polyurethane membrane does not eliminate the unallowable air gap between the membrane and the end face of the distal part of the optical fiber probe. That is why, when using this protector it is necessary to place a drop of liquid gel or other fluid with an appropriate refraction index onto the inner surface of the protector window before placing the optical fiber probe inside the working space. The fluid fills up the air gap, the later allowing for minimizing the optical loss and eliminating artifacts in obtained images. However, this procedure is time consuming and fairly laborious, especially taking into account that the diameter of the protector may be 3 mm or less. Applying this fluid to the inner surface of the output window in the course of its manufacturing is impractical since it is impossible to retain the fluid on the surface in storage and shipping.

A protector for an optical fiber probe used for studying biological tissues described in U.S. Pat. No. 6,383,209 overcomes the above-identified drawback. This protector comprises a hollow flexible sheath with a closed distal end and an open proximal end. The flexible sheath allows for an optical fiber probe to be placed inside of it. The end face of the flexible sheath comprises a window which is at least partially optically transparent.

In a preferred embodiment the window is made of quartz. The sheath includes a fluid chamber placed in the distal end of the sheath and an inflation channel for delivering fluid to the chamber. The fluid filling the chamber provides an efficient optical contact between the end face of the distal part of the optical fiber probe and the protector window and hence provides an efficient optical contact between the end face of the distal part of the optical fiber probe and the biological tissue.

A disadvantage of this protector is its implementation complexity subject to the fact that the protector must be equipped with a fluid supply and with a device for delivering fluid into the chamber. The later, especially when using the protector for a miniature optical fiber probe with a diameter of about 3 mm or less, intended for endoscopic studies, is an extremely complicated task. Besides, the requirements for durability and pliability of the material the fluid chamber is made of are fairly severe, as well as for the material of the inflation channel. Another disadvantage of this prior art protector is that it does not allow to increase the friction coefficient between the outer surface of the protector window and the biological tissue. The later might occur necessary for preventing the protector window from sliding over a hard surface, for example, when studying teeth.

## Summary of the invention

The present invention is directed to a protector design for an optical fiber probe that ensures an effective optical contact between an end face of a distal part of an optical fiber probe and a biological tissue. The protector of the invention provides a temporary mechanical contact between the protector window and the biological tissue, it is technologically effective and can be implemented without using complex design solutions.

The developed protector for an optical fiber probe, similarly to that known from U.S. Pat. No. 6,383,209 comprises a hollow sheath having a closed distal end and an open proximal end, the sheath allowing for an optical fiber probe to be placed inside the sheath, the closed distal end of the sheath being made as a protector window, and the protector window being at least partially optically transparent.

Unlike the known protector, according to the invention an inner surface of the protector window is designed capable of forming a temporary adhesive contact with an end face of a distal part of the optical fiber probe under a pressure of an axial force exerted on the optical fiber probe placed inside the sheath.

In a particular embodiment an outer surface of the protector window is designed capable of forming a temporary adhesive contact with the object being studied under the pressure of the axial force exerted on the optical fiber probe placed inside the sheath.

It is preferable to manufacture the protector window from a pliable and resilient material.

It is desirable for the refractive index of the material of the protector window at the operating wavelength to be defined by the following relation:

$$N_a \cong (N_b * N_c)^{1/2},$$

where

$N_a$ is the refractive index of the material of the protector window;
$N_b$ is the refractive index of the object being studied;
$N_c$ is the refractive index of the material of the distal part of the optical fiber probe.

In a specific embodiment the refractive index of the material of the protector window, the refractive index of the object being studied, and the refractive index of the material of the distal part of the optical fiber probe have substantially equal values at the operating wavelength.

It is appropriate to manufacture the protector window from a cured optical gel.

In a specific embodiment the protector window may be manufactured from a jelly-like material.

In another specific embodiment the protector window may be manufactured from a rubber-like material.

In another particular embodiment the protector window is configured as at least a bilayer structure, whereas at least the layer, one of whose surfaces makes the inner surface of the protector window, is manufactured from a pliable and resilient material.

In another particular embodiment the layer, one of whose surfaces makes the outer surface of the protector window, manufactured from a pliable and resilient material.

It is preferable that the refractive indexes of the materials of the layers configuring the protector window, the refractive index of the object being studied, and the refractive index of the material of the distal part of the optical fiber probe have substantially equal values at the operating wavelength.

It is desirable that at least the layer, one of whose surfaces makes the inner surface of the protector window, is manufactured from a cured optical gel.

In a particular embodiment at least the layer, one of whose surfaces makes the inner surface of the protector window, is manufactured from a jelly-like material.

In another particular embodiment at least the layer, one of whose surfaces makes the inner surface of the protector window, is manufactured from a rubber-like material.

In another embodiment at least the layer, one of whose surfaces makes the outer surface of the protector window, is manufactured from a cured optical gel.

In a specific embodiment at least the layer, one of whose surfaces makes the outer surface of the protector window, is manufactured from a jelly-like material.

In another specific embodiment at least the layer one of whose surfaces makes the outer surface of the protector window, is manufactured from a rubber-like material.

In another embodiment the protector window is made as a pliable membrane.

In another embodiment the proximal end of the sheath is interfaced with a distal end of a hollow handle, whereas the interior cavities of the sheath and the handle form a common working space for placing the optical fiber probe inside the working space.

It is desirable to have the handle equipped with a locking means for fixing the position of the optical fiber probe inside the working space.

In another embodiment the hollow sheath is of a tubular shape.

In another embodiment the cavity of the handle is of a tubular shape.

In another embodiment the hollow sheath is made pliable.

In another embodiment the hollow sheath is made rigid.

In another embodiment the optical fiber probe is part of a spectral imaging device.

In another embodiment the optical fiber probe is part of a device for optical coherence tomography.

In another embodiment the hollow sheath is manufactured from a material that is at least partially optically transparent.

It is desirable to make the protector reusable.

It is preferable to make the protector disposable.

In one embodiment the object being studied is a biological tissue.

In a specific embodiment the object being studied is a biological tissue of a living body.

In another specific embodiment the object being studied is a soft biological tissue of a living body.

In another specific embodiment the object being studied is a hard biological tissue of a living body.

In another specific embodiment the object being studied is an internal cavity of a living body.

The invention presents modifications of a protector design for an optical fiber probe intended for studying an object. The object being studied can be a biological tissue, namely, a biological tissue of a living body, for example, an internal cavity of a living body. The modifications of the protector design of the invention ensure an effective optical contact between the end face of the distal part of the optical fiber probe and the object being studied. In one modification the later is achieved by designing the inner surface of the protector window capable of forming a temporary adhesive contact with the end face of the distal part of the optical fiber probe under a pressure of an axial force exerted on the optical fiber probe placed inside a sheath. In another modification in addition to that, the outer surface of the protector window is designed capable of forming a temporary adhesive contact with the object being studied under the pressure of the axial force exerted on the optical fiber probe placed inside the sheath. To accomplish this in one embodiment the protector window is made of a pliable and resilient material, for example, of a cured optical gel. In another embodiment the protector window is configured as at least a bilayer structure. In one case, at least the layer, one of whose surfaces makes the inner surface of the protector window, is manufactured from a pliable and resilient material. In another case, additionally the layer, one of whose surfaces forms the outer surface of the protector window, is manufactured from a pliable and resilient material, such as a cured optical gel. This provides a temporary mechanical contact between the outer surface of the protector window and the object being studied. The later prevents the protector window from sliding over the surface of the object being studied and at the same time ensures an effective optical contact between the end face of the distal part of the optical fiber probe and the object being studied. The cured optical gel can be, e.g., jelly-like or rubber-like. Herewith, the value of the refractive index of the protector window material at the at the operating wavelength is chosen taking into account the values of the refractive indexes of the distal part of the optical fiber probe and of the object being studied. In a particular embodiment the protector may be interfaced with a handle, which can be equipped with a locking means for fixing the position of the optical fiber probe. Particular shapes and types of the sheath and handle, belonging of the optical fiber probe to one or another optical device, the same as designation of the object being studied, characterize the invention in its particular specific embodiments.

## Brief Description of Drawings

The features of the invention will be apparent from the following detail description of preferred embodiments with reference to the accompanying drawings, in which:

Fig. 1 is a cross-sectional view of a particular embodiment of the developed protector for an optical fiber probe.

Fig. 2 is a cross-sectional view of another particular embodiment of the developed protector for an optical fiber probe.

## Detailed Description of the Invention

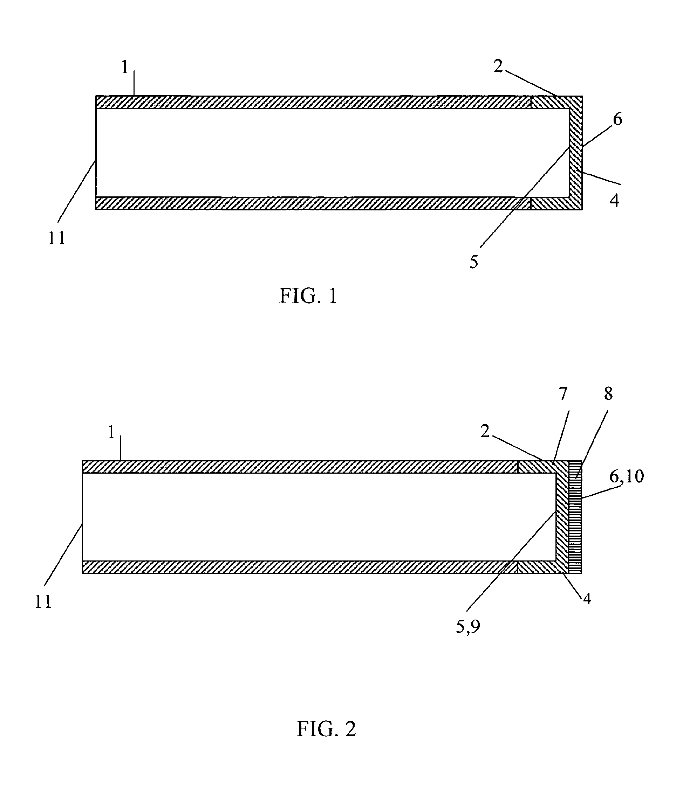

The protector shown in Fig. 1 operates as follows.

An optical fiber probe (not shown in the drawing) is placed inside a sheath 1, which is designed to allow for the optical fiber probe to be placed inside of it. A closed distal end 2 of the sheath 1 is made as a window 4, which is at least partially, optically transparent. An end face of a distal part of the optical fiber probe is brought into contact with an inner surface 5 of the window 4. The inner surface 5 of the window 4 is designed capable of forming a temporary adhesive contact with the end face of the distal part of the optical fiber probe under a pressure of an axial force exerted on the optical fiber probe placed inside the sheath 1. An outer surface 6 of the protector window 4 is designed capable of forming a temporary adhesive contact with an object being studied (not shown in the drawing) under the pressure of the axial force exerted on the optical fiber probe placed inside the sheath 1.

The window 4 is manufactured from a pliable and resilient material, such as, e.g. cured optical gel of the Smartgel type, which is produced by NYE (USA). The cured optical gel can be jelly-like or rubber-like. Herewith, it is desirable for the refractive index of the material of the protector window 4 at the operating wavelength to be defined by the following relation:

$$N_a \cong (N_b * N_c)^{1/2},$$

where

$N_a$ is the refractive index of the material of the protector window;
$N_b$ is the refractive index of the object being studied;
$N_c$ is the refractive index of the material of the distal part of the optical fiber probe.

In a specific embodiment the refractive index of the material of the protector window 4, the refractive index of the object being studied, and the refractive index of the material of the distal part of the optical fiber probe at the operating wavelength may have substantially equal values.

The protector with the optical fiber probe placed inside of the sheath 1 is positioned in a way to ensure delivery of optical radiation to the object being studied. In a specific embodiment when the optical fiber probe is an endoscopic probe, the protector is placed in a way that the outer surface 6 of the window 4 lies in direct contact with the object being studied. The pressure of the axial force is then exerted on the optical fiber probe, which forms a temporary adhesive contact between the inner surface 5 of the window 4 and the end face of the optical fiber probe. At the same time an adhesive contact is formed between the outer surface 6 of the window 4 and the object being studied that ensures a mechanical and effective optical contact between the window 4 and the object under study. Hence an effective optical contact is ensured between the end face of the optical fiber probe and the object being studied. After the study session is completed the axial force is relieved from the optical fiber probe. Since the adhesive contacts between the inner surface 5 of the window 4 and the end face of the optical fiber probe are temporary, as well as between the outer surface 6 of the window 4 and the object being studied, so after the axial force is relieved from the optical fiber probe the protector window is easily removed from the object being studied, and the probe is withdrawn from the sheath 1 with no trouble. Then the sheath 1 is sterilized or disinfected either disposed.

The protector shown in Fig. 2 operates analogous to that shown in Fig. 1. The design of the protector of Fig. 2 differs from the design of the protector of Fig. 1 by the window 4 being configured as at least a bilayer structure. In the particular embodiment the window includes a layer 7 and a layer 8. The layer 7 is manufactured from a pliable and resilient material. One of the surfaces of the layer 7, namely surface 9, makes the inner surface 5 of the window 4. The layer 8 is also manufactured from a pliable and resilient material. One of the surfaces of the layer 8, namely the surface 10, makes the outer surface 6 of the window 4. Cured optical gel of the Smartgel type produced by NYE (USA), which can be jelly-like or rubber-like can be used, for example, as a material for the layer 7 and the layer 8.

In a particular embodiment the refractive indexes of the materials of the layers 7, 8 configuring the protector window 4, the refractive index of the object being studied, and the refractive index of the material of the distal part of the optical fiber probe have substantially equal values at the operating wavelength.

In other respects, the protector for an optical fiber probe according to Fig. 2 is designed the same as the protector shown in Fig. 1.

In both designs the protector window 4 may be made as a pliable membrane. In both designs a proximal end 11 of the sheath 1 may be interfaced with a distal end of a hollow handle. In this case the interior cavities of the sheath 1 and the handle (not shown in the drawing) form a common working space for placing the optical fiber probe inside the working space. The handle may be implemented analogous to that described in U.S. Pat. No. 5,930,440 and may be equipped with a locking means for fixing the position of the optical fiber probe inside the working space (not shown in the drawing). The locking means may be analogous to that described in U.S. Pat. No. 5,930,440. The hollow sheath, the same as the cavity of the handle may be made tubular. In both protector designs, depending on the field of use the hollow sheath 1 may be made pliable, for example, of polyurethane. Or it could be made rigid, for example, of plastic. The material, from which the sheath 1 is made, could be optically opaque or at least partially optically transparent. The protectors, shown in Fig. 1 and in Fig. 2, can be made reusable (in this case they must be cleaned and disinfected for each patient) or disposable. The object being studied may be a biological tissue, for example, a biological tissue of a living body. It can be a hard tissue, such as teeth, or a soft tissue, such as an internal cavity of a living body. The optical fiber probe for which the various embodiments of the protector are designed, may be part of a spectral imaging device, or part of a device for optical coherence tomography, or be part of any endoscopic equipment.

**Industrial Applicability**

The invention can be utilized, for example, in low coherence reflectometers, in optical coherence tomography devices, in spectral imaging devices used for medical diagnostics of individual organs and systems of human body in vivo and in vitro, as well as for industrial diagnostics, such as control of technological processes. It should be noted that the invention may be implemented with the aid of standard facilities.

**Claims**

1. A protector for an optical fiber probe designed for studying an object comprising a hollow sheath having a closed distal end and an open proximal end, the sheath allowing for an optical fiber probe to be placed inside the sheath, the closed distal end of the sheath being made as a protector window, and the protector window being at least partially optically transparent, **characterized in that** an inner surface of the protector window is designed capable of forming a temporary adhesive contact with an end face of a distal part of the optical fiber probe under a pressure of an axial force exerted on the optical fiber probe placed inside the sheath.

2. The protector according to claim 1, **characterized in that** an outer surface of the protector window is designed capable of forming a temporary adhesive contact with the object being studied under the pressure of the axial force exerted on the optical fiber probe placed inside the sheath.

3. The protector according to claim 1 or claim 2, **characterized in that** the protector window is manufactured from a pliable and resilient material.

4. The protector according to claim 3, **characterized in that** the refractive index of the material of the protector window at the operating wavelength is defined by the following relation:

$$N_a \cong (N_b * N_c)^{1/2},$$

where

$N_a$ is the refractive index of the material of the protector window;
$N_b$ is the refractive index of the object being studied;

$N_c$ is the refractive index of the material of the distal part of the optical fiber probe.

5. The protector according to claim 3, **characterized in that** the refractive index of the material of the protector window, the refractive index of the object being studied, and the refractive index of the material of the distal part of the optical fiber probe have substantially equal values at the operating wavelength.

6. The protector according to claim 3, **characterized in that** the protector window is manufactured from a cured optical gel.

7. The protector according to claim 3, **characterized in that** the protector window is manufactured from a jelly-like material.

8. The protector according to claim 3, **characterized in that** the protector window is manufactured from a rubber-like material.

9. The protector according to claim 1 or claim 2, **characterized in that** the protector window is configured as at least a bilayer structure, whereas at least the layer, one of whose surfaces makes the inner surface of the protector window, is manufactured from a pliable and resilient material.

10. The protector according to claim 9, **characterized in that** the layer, one of whose surfaces makes the outer surface of the protector window, is manufactured from a pliable and resilient material.

11. The protector according to claim 9, **characterized in that** the refractive indexes of the materials of the layers configuring the protector window, the refractive index of the object being studied, and the refractive index of the material of the distal part of the optical fiber probe have substantially equal values at the operating wavelength.

12. The protector according to claim 9, **characterized in that** at least the layer, one of whose surfaces makes the inner surface of the protector window, is manufactured from a cured optical gel.

13. The protector according to claim 9, **characterized in that** at least the layer, one of whose surfaces makes the inner surface of the protector window, is manufactured from a jelly-like material.

14. The protector according to claim 9, **characterized in that** at least the layer, one of whose surfaces makes the inner surface of the protector window, is manufactured from a rubber-like material.

15. The protector according to claim 9, **characterized**

**in that** at least the layer, one of whose surfaces makes the outer surface of the protector window, is manufactured from a cured optical gel.

16. The protector according to claim 9, **characterized in that** at least the layer one of whose surfaces makes the outer surface of the protector window, is manufactured from a jelly-like material.

17. The protector according to claim 9, **characterized in that** at least the layer one of whose surfaces makes the outer surface of the protector window, is manufactured from a rubber-like material.

18. The protector according to claim 1 or claim 2, **characterized in that** the protector window is made as a pliable membrane.

19. The protector according to claim 1 or claim 2, **characterized in that** the proximal end of the sheath is interfaced with a distal end of a hollow handle, whereas the interior cavities of the sheath and the handle form a common working space for placing the optical fiber probe inside the working space:

20. The protector according to claim 19, **characterized in that** the handle is equipped with a locking means for fixing the position of the optical fiber probe inside the working space.

21. The protector according to claim 1 or claim 2, **characterized in that** the hollow sheath is of a tubular shape.

22. The protector according to claim 21, **characterized in that** the cavity of the handle is of a tubular shape.

23. The protector according to claim 1 or claim 2, **characterized in that** the hollow sheath is made pliable.

24. The protector according to claim 1 or claim 2, **characterized in that** the hollow sheath is made rigid.

25. The protector according to claim 1 or claim 2, **characterized in that** the optical fiber probe is part of a spectral imaging device.

26. The protector according to claim 1 or claim 2, **characterized in that** the optical fiber probe is part of a device for optical coherence tomography.

27. The protector according to claim 1 or claim 2, **characterized in that** the hollow sheath is manufactured from a material that is at least partially optically transparent.

28. The protector according to claim 1 or claim 2, **characterized in that** it is made reusable.

29. The protector according to claim 1 or claim 2, **characterized in that** it is made disposable.

30. The protector according to claim 1 or claim 2, **characterized in that** the object being studied is a biological tissue.

31. The protector according to claim 1 or claim 2, **characterized in that** the object being studied is a biological tissue of a living body.

32. The protector according to claim 1 or claim 2, **characterized in that** the object being studied is a soft biological tissue of a living body.

33. The protector according to claim 1 or claim 2, **characterized in that** the object being studied is a hard biological tissue of a living body.

34. The protector according to claim 1 or claim 2, **characterized in that** the object being studied is an internal cavity of a living body.

FIG. 1

FIG. 2

# EP 1 619 527 A1

## INTERNATIONAL SEARCH REPORT

International application No.
PCT/RU 2004/000144

| A. | CLASSIFICATION OF SUBJECT MATTER |
|---|---|

G02B 6/36, A61B 1/07

According to International Patent Classification (IPC) or to both national classification and IPC

| B. | FIELDS SEARCHED |
|---|---|

Minimum documentation searched (classification system followed by classification symbols)

G02B 6/00, 6/24, 6/36, A61B 1/00, 1/002, 1/005, 1/06-1/07, 17/34, A61N 5/06, A61B 5/00, 17/34

Documentation searched other than minimum documentation to the extent that such documents are included in the fields searched

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | US 5318024 A (MASSACHUSETTS INSTITUTE OF TECHNOLOGY) 07. 06. 1994, column 12 lines 21-30, figure 10G | 1-34 |
| A | US 6383209 B1 (BOSTON SCIENTIFIC CORPORATION) 07. 05. 2002, the abstract | 1-34 |
| A | US 6224543 B1 (ADROIT MEDICAL SYSTEMS, INC.) 01. 05. 2001, claims 1-6 | 1-34 |
| A | US 1727798 A1 (SARATOVSKY GOSUDARSTVENNY MEDITSINSKY INSTITUT) 23.04.1992, the abstract, the drawing | 1-34 |
| A | US 5771327 A (OPTICAL BIOPSY) 23. 06. 1998, the abstract | 1-34 |

☐ Further documents are listed in the continuation of Box C.   ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 21 July 2004 (21.07.2004) | 05 August 2004 (05.08.2004) |
| Name and mailing address of the ISA/ RU | Authorized officer |
| Facsimile No. | Telephone No. |

Form PCT/ISA/210 (second sheet) (July 1992)